# EUROPEAN PATENT SPECIFICATION

(11) **EP 0 544 802 B1**
(45) Date of publication and mention of the grant of the patent: **22.01.1997**
(21) Application number: 91916302.2
(22) Date of filing: 23.08.1991
(51) Int. Cl.: A01N 31/08, A01N 31/00

(54) **METHODS AND COMPOSITIONS FOR TREATING T-CELL MEDIATED DISEASES**
VERFAHREN UND ZUSAMMENSETZUNGEN FÜR DIE BEHANDLUNG DURCH T-ZELLEN VERMITTELTER KRANKHEITEN
PROCEDES ET COMPOSITIONS DE TRAITEMENT DE MALADIES INDUITES PAR LES LYMPHOCYTES T

(30) Priority: 23.08.1990 US 572085
(43) Date of publication of application: 09.06.1993
(73) Proprietor: NEW YORK UNIVERSITY, New York, NY 10016 (US)
(72) Inventor: MERUELO, Daniel, Scarborough, NY 10510 (US); LAVIE, Gad, Tenafly, NJ 07070 (US)
(74) Representative: Bergvall-Eftring, Stina Lena
(86) International application number: US9106209
(87) International publication number: WO9203049

(56) References cited:
- WO-A-89/01329
- US-A- 4 705 687
- US-A- 4 880 803
- US-A- 4 989 891
- BIOCHEMICAL AND BIOPHYSICAL RESEARCH COMMUNICATIONS, vol. 165, no. 3, 1989, pages 1207-1212; I. TAKAHASHI ET AL.: 'HYPERICIN AND PSEUDOHYPERICIN SPECIFICALLY INHIBIT PROTEIN KINASE C: POSSIBLE RELATION TO THEIR ANTIRETROVIRAL ACTIVITY'
- FARMATSIYA, vol. 34, no. 1, 1985, page 62-64; S.G. ZAICHIKOVA ET AL.: 'HEALING PROPERTIES AND DETERMINATION OF THE UPPER PARAMETERS OF TOXICITY OF HYPERICUM HERB'

## Description

### FIELD OF THE INVENTION

This invention pertains to the use of a composition comprising a compound selected from the group consisting of hypericin, pseudohypericin, an isomeric homolog, a homolog and a derivative of hypericin and pseudohypericin, and a pharmacologically acceptable carrier or diluent for the manufacture of a medicament for treating or preventing a T-cell mediated disease in a mammal as well as a pharmaceutical composition for treating or preventing a T-cell mediated disease.

### BACKGROUND OF THE INVENTION

T-cell mediated diseases are characterized by the induction of cytotoxic T-lymphocytes expressing the CD8 antigen on their cell surface and/or helper T-cells expressing the CD4 antigen on their cell surface. These diseases, exemplified by graft-versus-host disease, graft rejection, and a number of autoimmune disorders such as multiple sclerosis, rheumatoid arthritis, Graves disease, Addison's disease and scleroderma, represent a large number of host immune system disorders.

Graft-versus-host disease occurs when immune system cells, such as stem cells or lymphocytes from a donor, are transplanted into an allogenic host, i.e. one with different major histocompatibility (tissue compatibility) antigens. Immune system cells are transplanted into patients in order to treat certain forms of leukemia and/or for patients suffering from radiation exposure. The host is typically rendered immunologically silent (i.e. suppressed from mounting an immune response against the transplanted cells) by irradiation and the use of immunosuppressive drugs in order to make the host tolerant to the "foreign" tissue. In these diseases the transplanted immune system cells recognize the host as a foreign organism and mount an immune response directed against the host. The clinical manifestations of graft-versus-host disease in humans include fever, rash, anorexia, nausea, vomiting and watery or bloody diarrhea.

In addition, it has recently been reported that transfusion associated graft-versus-host disease can occur in immunocompetent transfusion recipients (Anderson, K.C., et al., New Eng. J. Med 323: 315-321, 1990)

Allogenic graft recipients, i.e. recipients of tissue grafts from members of the same species, are usually treated with cytotoxic drugs such as cyclosporin A (CsA), FK-506 (a derivative of CsA as disclosed in Metcalfe, S. et al., Transplantation (Baltimore) 49: 798-802, 1990), Cytoxan (cyclophosphamide) and recently a new drug, 15-deoxyspergualin (as disclosed in Amemiya, H., et al., Transplantation 49: 337-343, 1990). These drugs suppress the immune response of the host against the transplanted tissue and thereby help to prevent graft rejection. CsA has been found to inhibit the activation of T-cells by inhibiting the production and/or the secretion of lymphokines and monokines such as interleukin-1, interleukin-2, tumor necrosis factor (TNF) and gamma-interferon, substances which are involved in the activation of cytotoxic T-cells. Use of CsA, cyclophosphamide, 15-deoxyspergualin and FK-506 is limited due to the cytotoxic effect of these drugs on the host and also because they induce "global" immunosuppression, i.e., prolonged immunosuppressive treatment with these drugs can lead to infections and malignancies. Indeed, CsA therapy has been found to cause the appearance of tumors, particularly lymphomas in the treated host necessitating discontinuance of CsA therapy. CsA is also suspected to cause diabetagenic changes in treated patients (Wahlstrom, M.E., et al., Transplantation 49: 600-604, 1990).

Other therapies for treating allograft rejection involve the administration of OKT-3 monoclonal antibodies directed against the T₃ receptor in the T₃T₂ complex located on the cell surface of cytotoxic T-cells (New Eng. J. Med. 313: 338, 1986). OKT-3 treatment eliminates the T-effector cells which mediate this reaction.

An autoimmune disease is a malfunction of the immune system of mammals, including humans. In a mammal afflicted with an autoimmune disease, the immune system cannot or does not distinguish between exogenous (foreign) antigens and normal (self) tissues. As a result the immune system recognizes autologous tissue (self antigens) and substances as if they were foreign and evokes the proliferative immune response usually reserved for use against exogenous (foreign) tissues or invading organisms.

Current treatments for autoimmune diseases also involve administration of drugs which non-specifically suppress the immune response. Examples of such drugs include methotrexate, Cytoxan (cyclophosphamide), Imuran (azathioprine), FK-506 and cyclosporin A. Steroid compounds, such as prednisone and methylprednisilone are also employed in many instances. These drugs have limited efficacy against autoimmune diseases, and as mentioned above, have toxic side effects and tend to induce a "global" immunosuppression in patients.

Another proposed therapy for treating autoimmune diseases involves oral administration of the antigen involved in the particular autoimmune disease, e.g. oral administration of collagen to treat collagen-induced arthritis is disclosed in Nagler-Anderson et al. (Proc. Nat. Acad. Sci USA 83: 7443-7446, 1986); oral adminstration of myelin basic protein to treat experimental allergic encephalomyelitis is disclosed in Higgins, P. et al., J. Immunol. 140:1440-1445, 1988. However, use of this therapeutic modality is limited due to the difficulty in identifying and purifying the antigen(s) responsible for the autoimmune disease that is afflicting the patient.

Hypericin and related polycyclic aromatic compounds have been used to treat a wide spectrum of diseases that are caused by viruses and retroviruses.

U.S. Patent No. 4,898,891 issued February 6, 1990, discloses the antiviral activity of two aromatic polycyclic dione compounds: hypericin (Hy) and pseudohypericin (Ps).

The assignees copending U.S. Patent Application Serial No. 328,767 filed March 20, 1989 as a continuation-in-part of U.S. Patent Application Serial No. 084,008 filed August 10, 1987 (abandoned) expands upon the disclosure of U.S. Patent No. 4,898,891, focusing on the use of Hy and Ps as effective anti-retroviral agents.

The assignees copending U.S. Patent Application Serial No. 326,392 filed March 28, 1989 as a continuation-in-part of U.S. Patent Application Serial No. 172,065 filed March 23, 1988 (abandoned) discloses antiretroviral compositions comprising effective amounts of Hy and Ps in combination with nucleoside analogs such as azidothymidine and methods for treating retroviral infections with such compositions.

The assignees copending U.S. patent application Serial No. 299,971, filed January 19, 1989 and PCT Application No. PCT/US90/00398 published July 21, 1990, discloses compositions and methods for inactivating viruses and retroviruses present in blood, blood products and other body fluids and, more generally biological fluids, as well as articles useful for practicing such methods. The compositions disclosed as being useful include hypericin, pseudohypericin, isomers, analogs, homologs, and derivatives of aromatic polycyclic diones and mixtures of these compounds, all of which are also used in the present invention.

The assignees copending U.S. Patent Application Serial No. 417,163 filed October 4, 1989 as a continuation-in-part of U.S. Patent Application Serial No. 324,177 filed March 19, 1989 (abandoned) and PCT application No. PCT/US90/04163 filed March 19, 1990, discloses compositions comprising aromatic polycyclic antiviral compounds and methods for treating viral infections. The disclosed polycyclic aromatic compounds include hypericin and related polycyclic diones as well as homologs, isomers, derivative, salts and analogs of such polycyclic dione compounds and mixtures thereof for treating viral and retroviral infections.

The assignees copending U.S. Patent Application Serial No. 552,889 filed May 14, 1990 discloses aqueous pharmaceutical formulations and compositions comprising water-dispersed polycyclic aromatic compounds with improved biological and/or physical properties and methods of use thereof in treating viral and retroviral diseases.

Heretofore, there has been no disclosure or suggestion of the effects of the polycyclic aromatic compounds on T-cells or on the immune system.

Thus, at the present time there are no effective agents and methods for treating T-cell mediated diseases that have low toxicity to the treated animals, but are effective in treating a broad spectrum of T-cell mediated diseases.

It is an object of the present invention to provide methods and pharmaceutical formulations useful for treating T-cell mediated diseases.

It is a further object of the present invention to provide methods and pharmaceutical formulations that have low toxicity for the host receiving treatment and are effective for treating T-cell mediated diseases.

### SUMMARY OF THE INVENTION

It has now been discovered that hypericin and related polycyclic aromatic compounds can be used to treat a wide variety of diseases which are mediated by T-cells. It is believed that the effectiveness of these compounds in treating T-cell mediated diseases is attributable to the ability of the compounds to interfere with some aspects of T-cell function.

In one aspect the present invention provides the use of a composition comprising a compound selected from the group consisting of hypericin, pseudohypericin, an isomeric homolog, a homolog and a derivative of hypericin and pseudohypericin, and a pharmacologically acceptable carrier or diluent for the manufacture of a medicament for treating or preventing a T-cell mediated disease in a mammal.

Another aspect of the present invention involves a pharmaceutical composition for treating or preventing a T-cell mediated disease, comprising a therapeutically effective amount of (a) at least one compound selected from the group consisting of hypericin, pseudohypericin, an isomeric homolog, a homolog and a derivative of hypericin and pseudohypericin; and (b) an amount of an immunosuppressive agent, wherein the combined amounts of (a) and (b) are effective to treat or prevent said T-cell mediated disease.

These and other aspects of the present invention will be apparent to those of ordinary skill in the art in light of the present description, claims and drawings.

### BRIEF DESCRIPTIONS OF THE DRAWINGS

Fig. 1 is a graph illustrating the effect of hypericin administered 3 times a week on graft-versus-host disease.

Fig. 2 is a graph illustrating the effect of hypericin on graft-versus-host disease in mice treated 3 times a week with hypericin measuring animal morbidity.

Fig. 3 A and B are a series of graphs showing a comparison of the efficiency in treating graft-versus-host disease in mice of administering hypericin one, two, three, four and five times per week.

Fig. 4 (A and B) are graphs illustrating the effects on graft-versus-host disease in mice of administering cyclosporin A, hypericin and the hypericin analogs WIS-3, WIS-6 and WIS-7, measuring animal morbidity.

Fig. 5 (A and B) are graphs illustrating the effects of administrating cyclosporin A, hypericin and the hypericin analogs WIS-3, WIS-6 and WIS-7 on graft-versus-host disease in mice, measuring animal survival.

### DETAILED DESCRIPTION OF THE INVENTION

All patents, patent applications and literature references mentioned in this specification are hereby incorporated by reference in their entirety.

As used herein, the term "treat" or "treatment" shall mean prophylactic administration to prevent disease or, therapeutic administration to treat an active T-cell mediated disease in an afflicted individual.

As employed herein the term "mammal" refers to all life forms that have an immune system and are therefore susceptible to T-cell mediated diseases.

As used herein the term "polycyclic aromatic compound" refers to hypericin, pseudohypericin, isomers, analogs, homologs and derivatives of polycyclic diones as disclosed in assignees copending U.S. Patent Application Serial No. 413,163 filed October 4, 1989 as a Continuation-in-part of U.S. Patent Application Serial No. 324,177 filed March 19, 1989 (abandoned) and PCT Application No. PCT/US90/01463 filed March 19, 1990.

As used herein the term "major histocompatibility complex" refers to a plurality of cell surface proteins and glycoproteins that are present on the surfaces of all mammalian cells and which mediate cell-cell interactions (e.g. tissue compatibility in organ and tissue transplantation) and the immune response in humans. The MHC is encoded in humans by the HLA complex and in mice by the H-2 complex.

It has now been discovered that hypericin and related polycyclic aromatic compounds can be used to treat a wide variety of diseases which are mediated by T-cells. This is a most surprising finding because there have been no previous published reports that hypericin and related polycyclic aromatic compounds have any effects on the immune system, and especially on T-cells.

The present invention can be used to treat a wide variety of diseases which are mediated by T-cells. Non-limiting examples of the diseases which may be treated with the present invention include graft-versus-host disease, graft rejection, and autoimmune diseases mediated by T-cells. Such diseases include by way of non-limiting example multiple sclerosis, rheumatoid arthritis, Addison's disease, Graves disease, scleroderma, polymyositis, certain forms of diabetes mellitus (such as Juvenile onset diabetes), autoimmune uveoretinitis, systemic lupus erythromatosis, ulcerative colitis, pemphigus vulgaris and autoimmune thyroiditis.

An autoimmune disease is a malfunction of the immune system of mammals, including humans. The immune system of a mammal afflicted with such a disease cannot or does not distinguish between exogenous (foreign) antigens and normal (self) tissues. As a result, the immune system recognizes autologous tissues (self antigens) and substances as if they were foreign materials and evokes the proliferative immune response usually reserved for use against exogenous (foreign) tissues or invading microorganisms.

The method of the present invention comprises administration to a mammal in need of such treatment of an effective amount to treat or prevent a T-cell mediated disease of a polycyclic aromatic compound (alternatively referred to hereinafter as a "PAC").

Preferred PAC's for use in the present invention include hypericin, pseudohypericin, hypericin diacetate (WIS-6), desmethyl hypericin (WIS-3), hypericin hexaacetate and dihydroxydesmethyl hypericin (WIS-7) and most preferably hypericin and pseudohypericin. All of the PAC's are useful in treating T-cell mediated diseases in mammals, however in particular individuals or diseases one PAC may be more effective. Identification and selection of the most effective PAC for treating a particular T-cell mediated disease can be determined using routine experiments and techniques that are well-known to those of ordinary skill in the art. These techniques include the use of suitable in vivo systems such as those set forth in Examples 1-5 below.

In addition, other experimental systems may be employed to optimize the method of the present invention. For example, the effect of the PAC's of the present invention on multiple sclerosis can be examined in the experimental allergic encephalomyelitis (EAE) system in mammals as disclosed in Higgins, J. et al., J. Immunol. 140:1440-1445, 1988. EAE is an induced T-cell-mediated autoimmune disease directed against myelin basic protein (MBP). EAE is a recognized and widely used animal model of the human disease multiple sclerosis. The disease is induced by parenteral administration of MBP and an adjuvant (such as Freund's complete adjuvant). This treatment induces either a monophasic or an exacerbating/remitting form of demyelinating disease (depending on the species of animal and details of administration) having the characteristics of the human disease multiple sclerosis.

In like manner, the effect of administering the PAC's and the PAC-containing compositions of the present invention to mammals afflicted with diabetes can be examined in the BB strain of mice or in the NOD strain of mice which develop diabetes spontaneously.

The effects of PAC and the compositions of the present invention on rheumatoid arthritis may be examined using the mouse model for the disease induced by parenteral administration of collagen as disclosed in Nagler-Anderson et al. (Proc. Nat. Acad. Sci. USA 86; 680-684, 1986).

Hypericin and related polycyclic aromatic compounds can be obtained as described in copending U.S. Patent Application Serial No. 413,163 filed October 4, 1989 as a continuation-in-part application of U.S. Patent Application Serial No. 324,177 filed March 19, 1989 (abandoned), PCT Application No. PCT/US90/04163 filed March 19, 1990, U.S. Patent Application Serial No. 299,971 and PCT Application No. PCT/US90/00398.

The PAC of the present invention can also be advantageously combined with one or more of the immunosuppressive agents that are commonly employed to treat mammals afflicted with T-cell mediated diseases. Due to the effectiveness of the PAC of the present invention on these diseases, it is contemplated that reduced amounts (by reduced amounts is meant quantities lower than those normally used when such agents are administered alone) of immunosuppressive agent(s) or alternative drug regimens can be employed when such agents are administered in combination with the PAC's of the present invention, thereby reducing their frequency of administration and also reducing the toxic side effects of these agents. Non-limiting examples of the immunosuppressive agents that can be used in the present invention include cyclosporin A (Sandoz Pharmaceuticals, East Hanover, NJ), Imuran (azathioprine, Burroughs Welcome, Research Triangle Park, NC), Cytoxan, (cyclophosphamide, Bristol Meyers Oncology, Evansville, IN), prednisone (Lederle Laboratories, Wayne, NJ), methylprednisilone (Duramed Pharmaceuticals, Cincinnati, OH) and OKT-3 monoclonal antibodies (Ortho Diagnostics, Raritan, NJ).

In addition, FK-506 can be obtained from the fermentation broth of Streptomyces tsukubaensis (commercially available from Fujisawa Pharmaceutical Co., Osaka, Japan) as described in Thomas, J. et al., (Transplantation 49: 390-396, 1990).

Effective treatment of a given T-cell mediated disease may be achieved by using a single one of such PAC compounds, or a combination of two or more of such compounds. Naturally, it is desirable to employ the smallest possible quantity of the PAC compounds and/or immunosuppressive agents of the present invention that will provide a significant treatment to the patient. What constitutes "significant treatment" varies from patient to patient and also from one disease to another. In addition, one, two or more of the PAC compounds can be used together. That is to say, two or more PAC's may be co-administered to a patient, or they may be sequentially administered. Moreover, the PAC or mixtures thereof may constitute the sole active ingredient of the composition of the present invention or the PAC may be administered in conjunction with other agents or ingredients (such as immunosuppressive agents as disclosed below) that are known to be active in treating the disease or otherwise ameliorating or abolishing the T-cell mediated disease or the symptoms of such a disease.

In one preferred embodiment the present invention provides a method for treating or preventing a T-cell mediated disease by administering to a mammal in need of such treatment an amount of (a) a PAC and (b) an immunosuppressive agent, the combined amount of (a) and (b) in combination being effective to treat or prevent the T-cell mediated disease.

Determination of the most effective compound or mixture of compounds for a use in treating a mammal afflicted or at risk for the particular T-cell mediated disease can be accomplished by routine experimentation using suitable and well known experimental models as described above.

When employed to treat a T-cell mediated disease, the pharmaceutical formulations of the present invention may be administered orally, topically or preferably parenterally, and most preferably intravenously at dosages which can be broadly defined by reference to hypericin as follows below.

The present invention also provides compositions or pharmaceutical formulations or dosage forms for treating or preventing a T-cell mediated disease. Compositions or pharmaceutical formulations comprising the PAC of the present invention as the sole, active ingredients can be used at dosages (based on hypericin) containing from about 0.001 micrograms to about 200,000 micrograms per kilogram body weight per treatment, preferably between about 2 micrograms and about 10⁵ micrograms per kilogram body weight per treatment, and most preferably between about 200 micrograms and 5 X 10⁴ micrograms per kilogram body weight per treatment. The frequency of administration can vary from daily, twice daily, 2-, 3-, 4- or 5-times a week depending upon the patient's condition, response to the treatment and the severity of the patient's disease.

When one or more PAC are used as the active ingredient, the broad dosages will generally be the same as with hypericin. It is understood, however, that if a given PAC has e.g. twice the activity of hypericin in treating a particular T-cell mediated disease, the minimum effective dosage will be one-half that of hypericin. Moreover, when more than one active ingredient is used in a therapeutic or prophylactic regimen according to the invention (i.e., at least one non-PAC agent is included), the minimum dosage of the PAC component (i.e., the PAC compound or compounds) of this regimen may be reduced.

Finally, when more than one active ingredient is used and there is synergism between the PAC component and the other ingredient or ingredients (or between two or more PAC compounds, even in single active ingredient regimens, i.e., in regimens where the only agent or agents used are PACs), the minimum effective PAC dosages will be even lower. It should be also understood that analogous minimum dosage modifications apply when a stabilizing or potentiating agent is used in conjunction with a PAC.

When one or more immunosuppressive agent is used in combination with the compounds of the present invention, the agent may be administered in conjunction with the PAC at doses dependent on the individual agent used. "In conjunction" is defined herein to mean contemporaneous administration or sequential administration, i.e. the PAC may be administered first and the immunosuppressive agent later, or vice versa.

For example, when cyclosporin A is used in conjunction with the PAC of the present invention, the cyclosporin may be administered to mammals at dosages broadly ranging between about 0.01 mg per kg and about 100 mg per kg body weight of said mammal per day. This may be less (in certain instances) than the normal cyclosporin dose which is between about 1 mg per kg and about 20 mg per kg of body weight per day for treating kidney graft rejection in a mammal. In like manner, azathioprine (Imaran) can be used at dosages broadly ranging between about 0.1 mg per kg and 20 mg per kg body weight of said mammal per day, while prednisone may be administered at dosages broadly ranging between about 0.1 mg per kg and 200 mg per kg body weight of said mammal per day. The amounts of these agents used can be determined and optimized using routine experimentation well-known in the art as described above.

The duration and number of doses or treatments required to control the disease will vary from subject to subject, depending upon the severity and stage of the illness and the subject's general condition and will also depend on the specific activity of each PAC and/or immunosuppressive agent as well as their toxicity (if any). The total dose required for each treatment may be administered in divided doses or in a single dose. The treatment may be administered daily, more than once daily, or two to five times a week, or as determined by the subject's condition and the stage of the disease. The treatments may be initiated before, substantially simultaneously with or after the onset of a T-cell mediated disease as defined below. For example, when treating a mammal who will undergo a tissue transplantation, the pharmaceutical formulations of the present invention may be administered 1-15 days before the transplantation as well as 1-360 days after the transplantation.

The PAC of the present invention can be incorporated in conventional, solid and liquid pharmaceutical formulations (e.g. tablets, capsules, caplets, injectable and orally administrable solutions) or locally by using suppositories for use in treating mammals that are afflicted with T-cell mediated diseases. The pharmaceutical formulations of the invention comprise an effective amount of the PAC of the present invention as one of the active ingredients in combination with an immunosuppressive agent as discussed above. For example, a parenteral therapeutic pharmaceutical formulation may comprise a sterile isotonic saline solution containing between about 0.001 micrograms and about 100,000 micrograms of the polycyclic aromatic compounds of the present invention and between about 0.01 mg per kg and 100 mg of the immunosuppressive agent as described above. It will be appreciated that the unit content of active ingredients contained in an individual dose of each dosage form need not in itself constitute an effective amount since the necessary effective amount can be reached by administration of a plurality of capsules, tablets, injections or combinations thereof.

The pharmaceutical formulations of the present invention may contain well known inert constituents including pharmaceutically-acceptable carriers, diluents, fillers, salts, and other materials well known in the art, the selection of which depends upon the dosage form utilized and the particular purpose to be achieved according to the determination of the ordinarily skilled artisan in the field. For example, tablets may be formulated in accordance with conventional procedures employing solid carriers well known in the art. Examples of solid carriers include carboxymethyl cellulose, starch, sugar, bentonite, silica and other commonly used carriers and enteric coatings. Propylene glycol, polyethylene glycol, benzyl alcohol, isopropanol, ethanol, dimethylsulfoxide (DMSO), dimethylacetamide or other biologically acceptable organic solvents and aqueous detergent solutions, such as 1-5% Tween 80, or aqueous solutions may be employed. Mixtures of aqueous solutions (e.g. water with a pH higher than 7 and preferably about 8 or 5% dextrose) with basic amino acids such as lysine or aqueous solutions containing Tris buffer and/or, 2-2.5% benzyl alcohol may be used as diluents, carriers or solvents in the preparation of solid and liquid pharmaceutical formulations containing the compositions of the present invention. Further non-limiting examples of carriers and diluents include carbohydrates, cyclodextrans, albumin and/or other plasma protein components such as low density lipoproteins, high density lipoproteins and the lipids with which these serum proteins are associated. Such lipids include phosphatidyl choline, phosphatidyl serine, phosphatidyl ethanolamine and neutral lipids such as triglycerides. Lipid carriers also include, without limitation, tocopherol and retinoic acid. Conventional liposomes, well known in the art, containing the formulations of the present invention may also be employed as a drug delivery system. Semisolid shaped formulations such as those well-known in the art (e.g. suppositories) are also contemplated for use in administering the active ingredients of the present invention.

Preferred parenteral dosage forms may comprise an aqueous solution comprising 2% benzyl alcohol, further diluted in 5% dextrose to a volume of 50 to 500 mls, containing between about 0.001 micrograms and about 100,000 micrograms of the polycyclic aromatic compounds of the present invention and between about 0.01 mg and 100 mg of an immunosuppressive agent.

Capsules employed in the present invention may be made from any pharmaceutically acceptable material, such as gelatin or cellulose derivatives. Sustained release oral and transdermal delivery systems may also be employed to administer the active ingredients of the present invention.

In the specific examples presented below, the effects of PAC on graft-versus-host disease (GVHD), graft rejection and the autoimmune disease EAE are disclosed. GVHD is a well recognized model system for T-cell mediated diseases.

In the GVHD system, C3H mice, which have the histocompatibility genotype H-2^{k}, were bred with BALB/c mice, having the genotype H-2^{d}. The F₁ offsprings produced by these matings have the genotype H-2^{d}/^{k}. The F₁ offsprings were then irradiated with a sublethal dose (450 rad) of gamma radiation to further weaken their immune system and were injected with immune system cells isolated from the spleens and lymph nodes of C3H mice. The injected cells repopulate the hematopoietic tissues, i.e. spleen, lymph node and thymus. The injected cells recognize the H2^{d} portion present on the cells of the F₁ offspring bearing the H2^{d/k} genotype as foreign and mount an immune response against the host mediated by cytotoxic T-lymphocytes (CTL). The CTL attack the host causing cachectia and vascular leakage leading to a ruffling of the animals fur, morbidity, weight loss and death within 20-40 days of transplantation. Injection of hypericin, at a dose of 150 micrograms per mouse, led to the survival of all mice in some experiments and most of the mice in other experiments in the treated groups while almost all of the mice which were not treated succumbed to GVHD and died within 20-40 days.

The present invention is described further below in specific working examples which are intended to illustrate the present invention without limiting the scope thereof.

### EXAMPLE 1

The effect of hypericin on graft-versus-host disease was examined in the experiments described below.

The experimental system which was utilized to measure graft-versus-host disease (GVHD) and to determine the effects of hypericin on this reaction involved the breeding of C3H/DiSN mice (Jackson Labs, Bar Harbor, ME) which carry a major histocompatibility locus (H-2) of the type H-2^{k} with BALB/c mice (Jackson Labs, Bar Harbor, ME) which are H-2^{d}, to obtain F₁ hetrozygous offspring which are H-2^{k}/^{d}. The F₁ hetrozygous offspring were used as graft recipients for parental cell grafts of immunocompetent cells derived from the spleen and bone marrow.

The F₁ offspring were irradiated with 450 Rad of cesium-137, providing radiation at 390 rads/minute, for 1.2 minutes. The damaged lymphoid organs of the irradiated mice were reconstituted by intravenous injection through the tail vein of 5 X 10⁶ cells or 2 X 10⁷ C3H immunocompetent cells derived from the spleen and bone marrow of the C3H mouse as described in Rappaport, H. et al., Amer. J. Pathol. 96:121-142, 1979. In this system, the recipient host mouse, (which carries the genotype C3H/BALB/c) recognizes the transferred C3H cells as self or non-foreign and cannot reject them. The grafted immunocompetent cells recognize the BALB/c-derived class I and class II antigens of the H-2^{d} cells as foreign antigens and elicit a cell-mediated immune response across these class I and class II major histocompatibility barrier. This reaction involves an initial large expansion (through cell proliferation) in the number of cells in these clones which are engaged in this reaction followed by maturation of specific cytotoxic lymphocytes capable of attacking and destroying cells which carry the H-2^{d} genetic allotype throughout the various organs of the grafted animal. This hyperstimulatory process of the immune system is also mediated by increased production and secretion of soluble lymphokines and monokines. These include the monokine interleukin-1 (IL-1), which causes fever and malaise and contributes to the production of other lymphokines such as tumor necrosis factor (TNF)-beta and-alpha and IL-2, the latter of which promotes lymphocyte proliferation. These substances elicit the processes of weight loss and runting known as cachectia which progressively exacerbate vascular leakage and the sickness of the animals, leading to ruffled fur and death at 100% rate within 20-40 days.

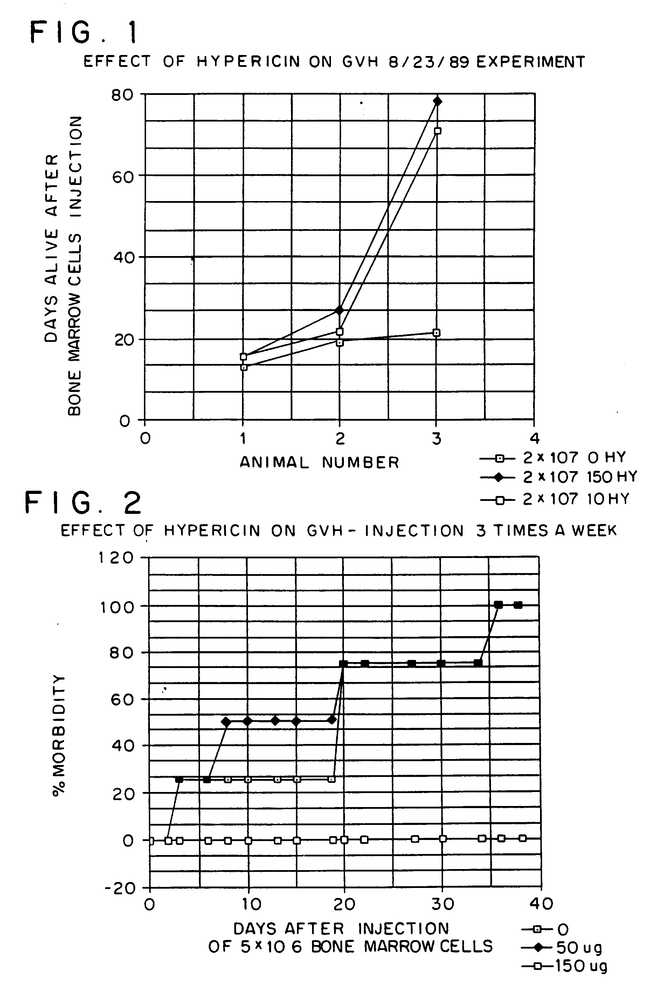

The transplant recipient mice were injected with hypericin intraperitoneally at doses of 10, 50 and 150 micrograms/mouse administered 3 times a week (every other day) beginning on day 8 after irradiation and cell inoculation. The results of this experiment are shown in Fig. 1.

In Fig. 1 it can be seen that at the cell inoculum used, 2 mice in the hypericin-treated group (one receiving 10 micrograms and one receiving 150 micrograms of hypericin) remained alive. Hypericin injections were discontinued 50 days after cell transfer and the surviving mice remained alive 21 additional days.

In a second experiment recipient mice were treated as above, inoculated with 2 X 10⁷ or 5 X 10⁶ cells and followed for 40 days post-transplantation. The results of these inoculations are depicted in Fig. 2 and set forth quantitatively in Table I.

In the data presented below in Table I, the morbidity observed in the inoculated mice was scored as follows:
- 0 =: mouse is healthy, its fur smooth, is active and escapes attempts to make physical contact with it;
- + =: mouse appears sick, its fur rough and its movements slow;
- 2+ =: pronounced lethargy, tends to remain stationary, and is easily picked up, remains motionless and does not attempt to escape;
- 3+ =: mouse assumes a hump back shape, weight loss evident, fur ruffled and the mouse confines itself to the corners of the cage without movement;
- 4+ =: weight loss and cachectic are pronounced, fur very ruffled and shivering is obvious; and
- D =: death

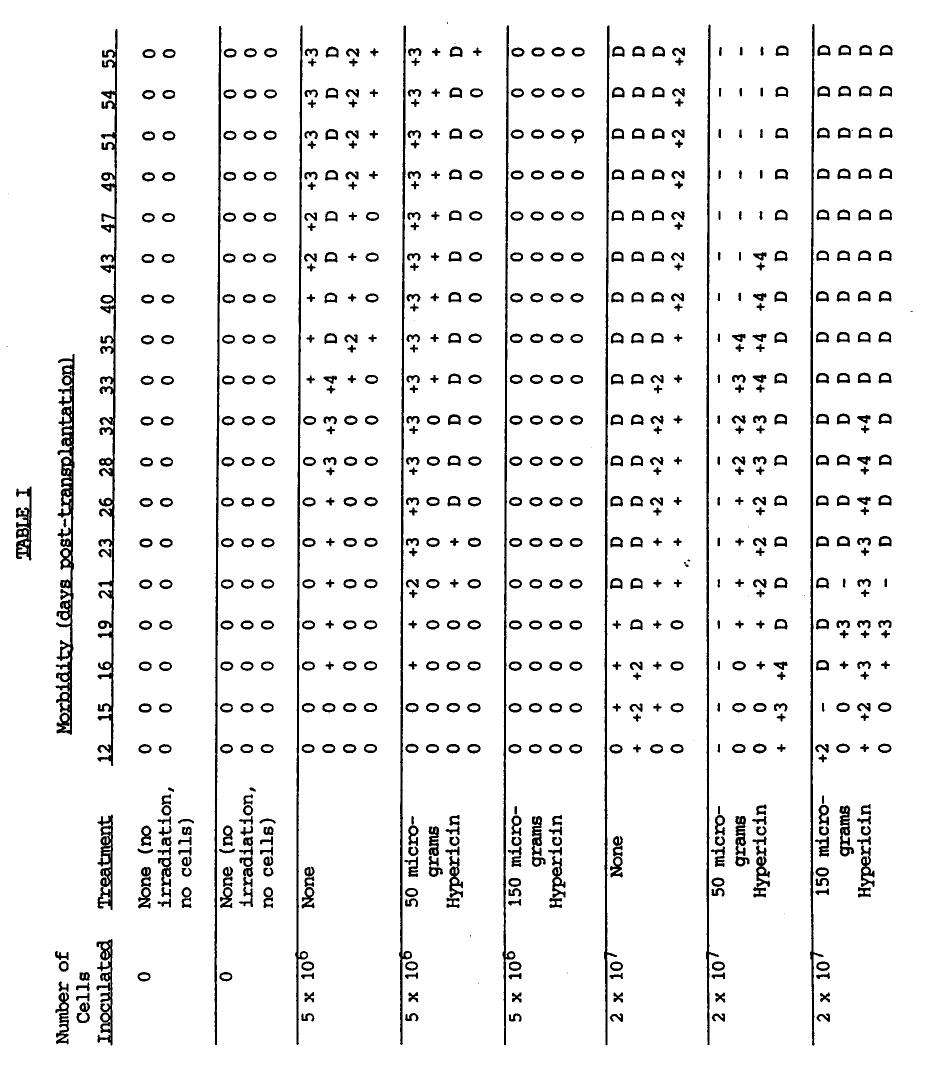

Table I and Fig. 2 show the results of morbidity and survival follow-upon these mice. In Fig. 2 and Table I it can be seen that at a cell inoculum of 5 x 10⁶ cells per mouse, all of the control mice began manifesting symptoms of GVHD as early as day 16 after transplantation and by day 35 after transplantation all of the control mice were affected (Table I). One mouse (out of 4), which received hypericin at 50 micrograms three times a week, was symptom-free until day 55 after-inoculation. However, all of the mice that received 150 micrograms of hypericin remained healthy throughout the entire experiment.

It should be noted that hypericin was ineffective, at doses of up to 150 micrograms per mouse used in these experiments, in both experiments when 2 x 10⁷ cells were used as the inoculum, perhaps due to the overwhelming burden of this inoculum. Further experiments are in progress employing higher concentrations of PACs with this cell inoculum.

This example established that hypericin was effective in lessening the symptoms of GVHD and prolonging the survival of treated animals.

### EXAMPLE 2

The effect of different frequencies of hypericin administration on acute graft-versus-host disease was analyzed in this experiment.

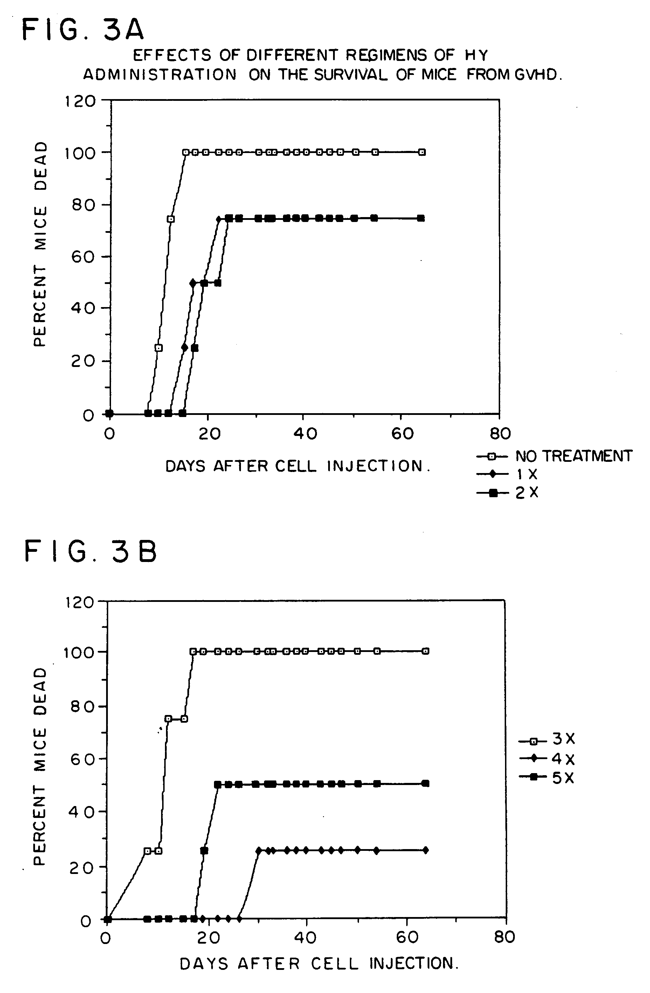

The same mouse strain system described above in Example 1 was used. 10⁷ C3H cells were injected into irradiated (C3H/BALB/c) F₁ mice. Eight days later, 150 micrograms/mouse of hypericin in 0.5 ml H₂O was injected intraperitoneally into the mice 0, 1X, 2X, 3X, 4X and 5X a week continuously up to day 70. The mice were then followed for survival over the 70 day follow-up period (Figures 3A and 3B).

As can be seen in Figs. 3A and 3B, only 1 of 4 mice injected with hypericin 4 times/week died within the 70 day follow-up period, and 2 of 4 mice were dead in the group which received 5 weekly injections. In those mice which died in this group, death occurred 15-20 days later than in the untreated, control group. All mice in the control group and in the group injected with hypericin 3 times/week were dead.

The results of this Example show that hypericin treatment, at a frequency of four and five times per week was more effective in treating GVHD than a three times a week regimen.

### EXAMPLE 3

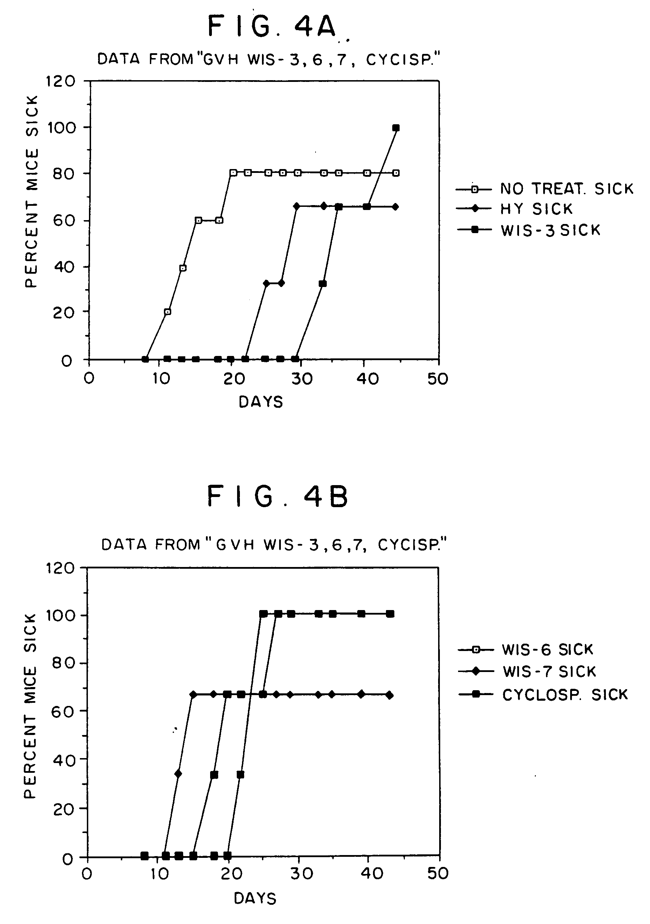

The effect of three hypericin analogs and derivatives, hypericin diacetate (WIS-6), desmethyl hypericin (WIS-3) and dihydroxydesmethyl hypericin (WIS-7) on the survival of mice suffering from GVH disease were studied in comparison to treatment with hypericin or cyclosporin A (obtained from Sandoz Pharmaceuticals, East Hanover, N.J.), the latter being one of the most effective treatments currently used to suppress T-cell mediated diseases. The experiments were conducted as described in the Examples 1 and 2 above but each of the drugs was injected intraperitoneally in water containing 0.5% ethanol at a dosage of 150 micrograms/mouse three times a week. The results of this experiment are shown in Fig. 4 (A and B) and Table II below.

**TABLE II**

| Morbidity (days post-transplantation) | | | | | | | | | | | | | | |
|---|---|---|---|---|---|---|---|---|---|---|---|---|---|---|
| Number of Cells Inoculated | Treatment | 8 | 11 | 15 | 18 | 21 | 22 | 25 | 28 | 32 | 36 | 39 | 41 | 46 |
| 0 | None (no irradiation, no cells) | 0 | 0 | 0 | 0 | 0 | 0 | 0 | 0 | 0 | 0 | 0 | 0 | 0 |
| | | 0 | 0 | 0 | 0 | 0 | 0 | 0 | 0 | 0 | 0 | 0 | 0 | 0 |
| | | 0 | 0 | 0 | 0 | 0 | 0 | 0 | 0 | 0 | 0 | 0 | 0 | 0 |
| 0 | None (no irradiation only) | 0 | 0 | 0 | 0 | 0 | 0 | 0 | 0 | 0 | 0 | 0 | 0 | 0 |
| | | 0 | 0 | 0 | 0 | 0 | 0 | 0 | 0 | 0 | 0 | 0 | 0 | 0 |
| | | 0 | 0 | 0 | 0 | 0 | 0 | 0 | 0 | 0 | 0 | 0 | 0 | 0 |
| 1 x 10⁷ | None | 0 | 0 | 0 | 0 | + | +2 | +3 | D | D | D | D | D | D |
| | | 0 | 0 | + | + | +2 | +3 | +3 | +3 | +3 | +3 | +3 | +3 | D |
| | | 0 | 0 | + | D | D | D | D | D | D | D | D | D | D |
| | | 0 | 0 | 0 | + | +3 | D | D | D | D | D | D | D | D |
| 1 x 10⁷ | 150 micrograms WIS-6 | 0 | 0 | 0 | 0 | + | + | D | D | D | D | D | D | D |
| | | 0 | 0 | + | D | D | D | D | D | D | D | D | D | D |
| | | 0 | 0 | 0 | 0 | + | + | +2 | D | D | D | D | D | D |
| | | 0 | 0 | 0 | 0 | +2 | D | D | D | D | D | D | D | D |
| 1 x 10⁷ | 150 micrograms WIS-3 | 0 | 0 | 0 | 0 | + | +2 | D | D | D | D | D | D | D |
| | | 0 | + | + | D | D | D | D | D | D | D | D | D | D |
| | | 0 | 0 | 0 | + | + | +2 | D | D | D | D | D | D | D |
| | | 0 | 0 | 0 | 0 | + | +2 | D | D | D | D | D | D | D |
| 1 x 10⁷ | 150 micrograms hypericin | 0 | 0 | 0 | 0 | 0 | 0 | 0 | 0 | 0 | 0 | 0 | 0 | 0 |
| | | 0 | 0 | 0 | 0 | 0 | 0 | 0 | 0 | 0 | 0 | 0 | 0 | 0 |
| | | 0 | 0 | 0 | + | D | D | D | D | D | D | D | D | D |

As can be seen by referring to Table II above, mice in the control groups (receiving irradiation and cells only) began manifesting symptoms of GVHD as early as 15 days post-transplantation. Three out of four of the mice in this group were dead by the 28th day after tissue-transplantation. WIS-3 and WIS-6 may have had a small effect in ameliorating GVHD symptoms. In contrast, 2 out of 3 hypericin-treated mice were healthy and showed no symptoms of GVHD throughout the entire 46 day follow-up period.

The results depicted in Figure 4 A and B show that treatment with WIS-3 (desmethyl hypericin) may prove to be superior to treatment with cyclosporin A. Both hypericin diacetate (WIS-6) and dihydroxydesmethyl hypericin (WIS-7) may have had some small effect in preventing or ameliorating GVHD as contrasted with the animals that received no treatment.

In Fig. 4A, 80% of the untreated mice were sick by day 46. Hypericin treatment lowered the number of sick mice to 60% and delayed the appearance of symptoms from day 8 (in the untreated group) until day 22. WIS-3-treated mice remained healthy until day 32 but rapidly became sick thereafter, perhaps due to the toxicity of WIS-3 at the dosage administered.

Fig. 4B shows that only 60% of the animals treated with WIS-7 were sick by day 46 post-transplantation whereas 100% of mice treated with WIS-6 or cyclosporin A were sick by day 46.

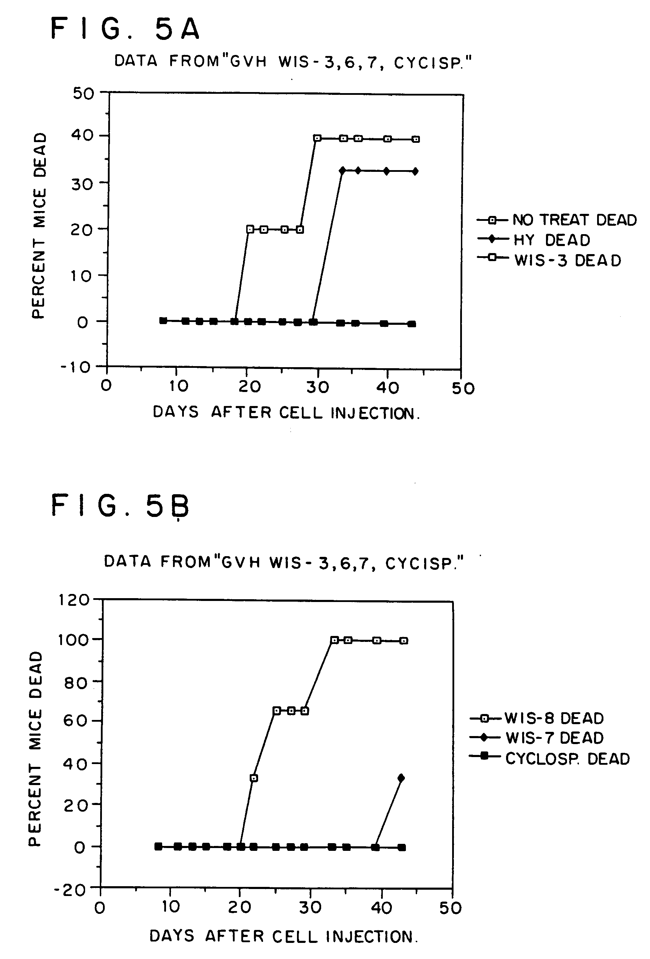

Fig. 5 (A and B) are graphs showing the survival of mice treated as described above. As can be seen in Fig. 5A, 40% of the mice which received no treatment (transplanted cells and irradiation only) were dead by day 46 whereas only 33% of the hypericin-treated group and 0% of the WIS-3 (desmethyl hypericin)-treated group were dead by day 46. In addition, hypericin-treated mice did not begin dying until day 30 versus day 17 in the control (non-treated) animals.

Fig. 5B shows that 0% of mice which received cyclosporin A were dead by day 46 whereas 100% of mice receiving WIS-7 and 33% of the mice which received WIS-7 were dead by day 46.

The above results show that hypericin, WIS-7 (and perhaps WIS-3) were more effective in treating GVHD than cyclosporin A.

### EXAMPLE 4

The effects of hypericin and related PACs on T-cell mediated skin allograft rejection in mice will be examined as described below.

C57BL/6 mice (Jackson Labs, Bar Harbor, ME) will be transplant recipients of tail skin from B10.A mice (Jackson Labs) using the procedure described in Billingham, R.E. et al. J. Exp. Biol. 28: 335, 1951. C57BL/6 mice carry a Major Histocompatibility genotype of H-2^{b} and have a class I genotype H-2K^{b} whereas B10.A mice differ in that they carry a H-2K^{d} genotype.

In this system, the skin graft is initially taken by the recipient C57BL/6 mice which develops vascularization to the grafted tissue. However, the immune system of the C57BL/6 mice recognizes the genetically disparate skin graft as foreign and mounts a T-cell mediated rejection reaction. The reaction severs the vasculature to the graft and the hosts' cytotoxic T-cells attack the cellular components of the grafted tissue leading to widespread damage to the grafted tissue. Approximately 20-22 days after transplantation the graft undergoes necrosis and is eliminated.

Hypericin, WIS-3, WIS-6 or WIS-7 will be administered three and five times per week beginning at day 8 post-transplantation at doses of 50, 100, 150 and 300 micrograms per mouse.

It is expected that the administration of hypericin and related PACs will result in a longer survival time for the grafted tissue beyond 70 days. Moreover, it is also expected that in the animals treated 5 times per week, graft survival will be extended beyond the survival times of grafts in animals receiving the PACs of the present invention 3 times per week.

### EXAMPLE 5

The effects of the PACs of the present invention on an autoimmune disease-experimental allergic encephalomyelitis (EAE, the animal model for the human disease multiple sclerosis) - will be studied in the following manner.

Guinea pig myelin basic protein (GP-MBP), obtained from guinea pig brain tissue (Pel Freeze Rogers, Arkansas) will be purified according to the method of Diebler, G.E. et al. (Anal. Biochem. 2: 139, 1972). Lewis rats, age 6-8 weeks (Charles River, Wilmington, MA) will be inoculated on day 0 with 10 micrograms of GP-MBP (obtained as above) in their foot pad in Freund's complete Adjuvant (Difco, Detroit, MI) as disclosed in Higgins, et al., J. Immunol. 140: 1440, 1988. The rats will be treated with various doses of hypericin, WIS-3, WIS-6 or WIS-7 three and five times a week and monitored for symptoms of EAE. Immunization of the rats to induce EAE on day 0 will result in an acute paralytic disease with symptoms manifesting on day 12 to 14 post-immunization. The rats will be scored using the following system: 0 = normal; 1 = loss of tail tone; 2 = weakness of back legs; 3 = paralysis of back legs; 4 = front leg weakness; 5 = moribund.

An additional set of animals will be pretreated with various doses of hypericin, WIS-3, WIS-6 or WIS-7 beginning 7-10 days before the induction of EAE on day 0 to determine the effects of these PAC on disease induction.

It is anticipated that the PAC of the present invention will be effective in both preventing the induction of EAE and ameliorating its symptoms.

## Claims

1. The use of a composition comprising a compound selected from the group consisting of hypericin, pseudohypericin, an isomeric homolog, a homolog and a derivative of hypericin and pseudohypericin, and a pharmacologically acceptable carrier or diluent for the manufacture of a medicament for treating or preventing a T-cell mediated disease in a mammal.

2. The use according to claim 1 of a compound selected from hypericin, pseudohypericin, desmethyl hypericin, hypericin diacetate and dihydroxydesmethyl hypericin.

3. A pharmaceutical composition for treating or preventing a T-cell mediated disease, comprising a therapeutically effective amount of (a) at least one compound selected from the group consisting of hypericin, pseudohypericin, an isomeric homolog, a homolog and a derivative of hypericin and pseudohypericin; and (b) an amount of an immunosuppressive agent, wherein the combined amounts of (a) and (b) are effective to treat or prevent said T-cell mediated disease.

4. A composition according to claim 3 wherein said immunosuppressive agent is selected from at least one of cyclosporin A, cyclophosphamide, methotrexate, prednisone, methylprednisilone, OKT-3, FK-506, 15-deoxyspergualine, and azathiorpine.

5. A composition according to claim 3 wherein said compound is selected from at least one of hypericin, pseudohypericin, desmethyl hypericin, hypericin diacetate, and dihydroxydesmethyl hypericin.

## Patentansprüche

1. Verwendung einer Zubereitung, umfassend eine aus der aus Hypericin, Pseudohypericin und einem isomeren Homologen, einem Homologen oder Derivat des Hypericins sowie des Pseudohypericin bestehenden Gruppe ausgewählte Verbindung und einen pharmakologisch annehmbaren Träger oder Verdünner, zur Herstellung eines Arzneimittels zur Behandlung oder Vorbeugung einer von T-Zellen vermittelten Erkrankung bei einem Säuger.

2. Verwendung gemäß Anspruch 1 einer Verbindung, die unter Hypericin, Pseudohypericin, Desmethylhypericin, Hypricindiacetat und Dihydroxydesmethylhypericin ausgewählt ist.

3. Pharmazeutische Zubereitung zur Behandlung oder Vorbeugung von T-Zell vermittelten Erkrankungen, welche eine therapeutisch wirksame Menge
(a) mindestens einer aus der aus Hypericin, Pseudohypericin und einem isomeren Homologen, einem Homologen oder Derivat des Hypericins sowie des Pseudohypericin bestehenden Gruppe ausgewählten Verbindung und
(b) eine Menge an einem Immunsuppressivum enthält,
wobei die vereinten Mengen an (a) und (b) bei der Behandlung oder Vorbeugung der T-Zell vermittelten Erkrankung wirksam sind.

4. Zubereitung gemäß Anspruch 3, bei der das Immunsuppressivum aus mindestens einem von Cyclosporin A, Cyclophosphamid, Methotrexat, Prednison, Methylprednisolon, OKT-3, FK-506, 15-Desoxyspergualin und Azathiorpin ausgewählt ist.

5. Zubereitung gemäß Ansspruch 3, bei der die Verbindung aus mindestens einem von Hypericin, Pseudohypericin, Desmethylhypericin, Hypericindiacetat und Dihydroxydesmethylhypericin ausgewählt ist.

## Revendications

1. Utilisation d'une composition comprenant un composé choisi dans le groupe qui consiste en hypéricine, pseudohypéricine, un homologue isomérique, un homologue et un dérivé d'hypéricine et de pseudohypéricine, et un véhicule ou diluant pharmacologiquement acceptable pour la fabrication d'un médicament pour traiter ou prévenir une maladie induite par les lymphocytes T chez un mammifère.

2. Utilisation selon la revendication 1 d'un composé choisi parmi l'hypéricine, la pseudohypéricine, la desméthyl hypéricine, le diacétate d'hypéricine et la dihydroxydesméthyl hypéricine.

3. Composition pharmaceutique pour traiter ou prévenir une maladie induite par les lymphocytes T, comprenant une quantité thérapeutiquement efficace (a) d'au moins un composé choisi dans le groupe qui consiste en hypéricine, pseudohypéricine, un homologue isomérique, un homologue et un dérivé d'hypéricine et de pseudohypéricine ; et (b) une quantité d'un agent immunodépresseur, dans laquelle les quantités combinées de (a) et (b) sont efficaces pour traiter ou prévenir ladite maladie induite par les lymphocytes T.

4. Composition selon la revendication 3, dans laquelle ledit agent immunodépresseur est choisi parmi au moins un des composés suivants : cyclosporine A, cyclophosphamide, méthotrexate, prednisone, méthylprednisilone, OKT-3, FK-506, 15-déoxyspergualine et aziathiorpine.

5. Composition selon la revendication 3, dans laquelle ledit composé est choisi parmi au moins un des composés suivants : hypéricine, pseudohypéricine, desméthyl hypéricine, diacétate d'hypéricine et dihydroxydesméthyl hypéricine.
